# EUROPEAN PATENT APPLICATION

(11) **EP 1 571 449 A1**
(43) Date of publication of application: **07.09.2005**
(21) Application number: 04075639.7
(22) Date of filing: 01.03.2004
(51) Int. Cl.: G01N 33/68, G01N 33/543

(54) **Prion immunoassay and separation method**

(71) Applicant: Stichting Sanquin Bloedvoorziening, 1066 CX Amsterdam (NL)
(72) Inventor: van Oers, Josephus, Wilhelmus, Alphonsus, Maria, 1506 TB Zaandam (NL); Hack, Cornelis, Erik, 1111 ND Diemen (NL); van Engelenburg, Franciscus, Antonius, Cornelis, 3555 EE Utrecht (NL)
(74) Representative: Winckels, Johannes Hubertus F.

(57) **Abstract**

Immunoassay method for detecting or measuring prion protein in a sample using at least one antibody specifically binding to prion protein, and a method of separating prion protein from a prion protein containing solution using a capturing antibody specifically binding to prion protein, comprising contacting the prion protein present with a reagent capable of improving binding of the antibody to the prion protein. The reagent may be a metal compound of a prion protein binding metal, especially a copper, nickel, zinc or manganese salt or chelate. The reagent may also be an oxidation agent, especially hydrogen peroxide or a permanganate salt. The invention also provides a method of determining the nature of prion protein which is present in a sample.

## Description

### Field of the Invention

This invention relates to the field of immunoassays for determining the presence or amount of prion protein (PrP) in a sample and to the field of diagnosis of prion diseases. The invention also relates to the field of separating PrP from a solution containing the same, to purify the solution from PrP or to collect PrP, and to the field of determining the nature of prion protein which is present in a sample.

This invention provides a specific improved PrP detection method, applicable for different anti-prion antibodies and in different immunoassay methods for prions, including ELISA, the invention resulting in at least a 3000- to 5000-fold increase in sensitivity. The technique disclosed herein is quick, easy, applicable on serum, plasma, cerebro spinal fluid (CSF), and urine samples and can be used on brain homogenates. Furthermore, it can be used with various labels.

Hence, this invention will be useful for diagnosis of transmissible spongiform encephalopathies in animal and human prion diseases, as well as diagnosis of other neurological diseases.

### Background of the invention

This invention relates to prion diseases, also called transmissible spongiform encephalopathies (TSE), and to methods to diagnose TSE in tissue or biological samples. Prion diseases or TSE are a group of fatal non-inflammatory neurodegenerative disorders examplified by bovine spongiform encephalopathy (BSE) or mad cow disease in cattle, scrapie in sheep, and Creutzfeldt-Jakob Disease (CJD) in man [18].

Prion diseases or TSE are infectious diseases, supposedly transmitted by a conformer of the naturally present prion protein (PrP), though cases may also occur spontaneously or from a genetic predisposition. Diagnosis of TSE typically requires demonstration of this conformer (PrP^{Sc}) in animal or human brain. Also elevated levels of normal PrP (PrP^{C}) in plasma are considered as a possible marker for a series of neurological diseases including TSE [27]. Detection of PrP in biological fluids is possible, though cumbersome due to a combination of interactions with other proteins, matrix effects and low concentrations.

TSE are characterised by a relatively long incubation period (e.g. >10 years in human), and typically share accumulation of PrP^{Sc} in brain and in some cases also in lymphoid tissues [22;29].

The prion protein is an endogenous protein, that also under normal conditions is expressed in the brain and various other tissues. This normal cellular prion protein (PrP^{C}) and the infectious variant thereof, i.e. PrP^{Sc}, are different conformations of the same protein. PrP^{C} and PrP^{Sc} differ in some physico-chemical and biochemical properties: PrP^{C} is soluble, exists in a non-aggregated form, and is sensitive to degradation by proteases, whereas PrP^{Sc} is insoluble, exists in an aggregated form, and is relatively resistant to degradation by proteases [16]. It is hypothesised that a direct protein-protein contact between PrP^{Sc} and PrP^{C} is needed to generate a conformational change of PrP^{C} into PrP^{Sc}. Ultimately PrP^{Sc} forms aggregates, accumulates in brain, and causes degeneration of neurons. At the final stage of the disease these neurodegerative changes cause severe neurological dysfunction, and ultimately death.

Copper-binding properties of PrP^{C} have been shown [2;8;9;25;27] and octarepeats, present in the N-terminal domain of PrP, are believed to be involved in this binding [8;9]. These findings suggest a possible role for PrP in copper metabolism. Copper ions serve as cofactors for many enzymes and, in particular, of energy-associated systems [6;17]. In addition, copper has also been shown to be toxic when present in excess, and therefore, an optimal copper concentration is required for good health [4;7;19]. The putative role of PrP in copper metabolism can vary from a copper transporter to a suppressor of copper-induced cell damage. The suppressor role has been shown in two copper-toxicity studies with wild-type (wt) and PrP knock-out mice: cells from the latter were more susceptible to copper toxicity than cells from wt mice, as has been shown for brain cells [1] and mature sperm cells [23]. Besides copper also Ni, Zn and Mn have been shown to bind PrP^{C} with high affinities [12].

Not only PrP, but also other proteins bind copper, especially a group of so-called S100 proteins. These proteins are present in CSF or plasma during TSE infection [5], and protect against copper-induced cellular damage [24].

Thackray et al. [26] described anti-PrP antibodies having 2- to 3-fold increased reactivity for metal-ion refolded recombinant PrP^{C}. The authors raised mAbs against copper refolded recombinant PrP^{C} and found that some antibodies with epitopes located in the carboxy-terminal region (a.a. 160 -231) have a better binding with copper refolded recombinant PrP^{C}, whereas antibodies against the N-terminal region did not show this effect.

PrP gene of various animal species has been cloned and sequenced [30]. The protein is encoded within a single exon, and consists of a long N-terminal signal sequence followed by about 250 amino acid (AA) residues. The C-terminus contains a sequence of about 22 residues coding for a linker site for a glycosyl-phosphatidylinositol anchor, a property shared with many other membrane proteins. Furthermore, the protein contains one disulfide bridge and two potential asp-glycosylation sites.

The sequence of the protein is markedly conserved among various animal species. Though differences in the secundary structure of PrP^{Sc} and that of PrP^{C} have been identified, the structural basis for the enhanced aggregatibility and proteolytic resistance of the PrP^{Sc} conformer as compared to PrP^{C}, is still not resolved. In addition, the molecular basis for the existence of different strains of PrP^{Sc}, i.e. prions with different incubation times, is lacking, though it is assumed that these strains may represent PrP^{Sc} with subtle differences in conformation [20].

Crucial for the diagnosis of TSE is detection of PrP^{Sc} in brain, using e.g. immuno-histochemistry or Western blotting. ELISA is becoming more and more popular in this field. For the in vitro diagnostic methods, polyclonal and monoclonal antibodies are usually applied [13;15]. Antibodies against prions described in the literature so far, do not discriminate between PrP^{C} and PrP^{Sc}, except for one that is however not suitable for regular immuno-diagnostics [14]. Because most antibodies bind to both conformers, the specificity of diagnostic methods is based on differences in resistance to proteinase K (PK) digestion, solubility, or degree of aggregation.

Detection of PrP^{Sc} in biological samples that can be obtained more easily than brain tissue, preferably blood, would also add to the diagnosis of TSE. However there is only sparse data, obtained with bioassays, available suggesting that PrP^{Sc} is present in blood [3;10;11;21].

To date, immunological evidence for the presence of PK-resistant, infectious prion proteins in plasma samples of TSE infected animals or human is lacking. Whether this reflects the extreme low concentrations of circulating PrP^{Sc} or problems associated with protease digestion of the various circulating prion proteins, remains unclear.

Alternatively detection of elevated concentrations of total prion proteins (PrP^{C} + PrP^{Sc}) in plasma has been suggested to be a surrogate marker for various neurological diseases, including CJD, alzheimer and MS [28].

### Summary of the invention

This invention provides an immunoassay method for detecting or measuring prion protein in a sample using at least one antibody specifically binding to prion protein, comprising contacting the prion protein present with a reagent capable of improving binding of said at least one antibody to the prion protein. Said at least one antibody may be a detection antibody, a catcher antibody, or both.

The invention also provides a method of separating prion protein from a prion protein containing solution using a capturing antibody specifically binding to prion protein, comprising contacting the prion protein present with a reagent capable of improving binding of the capturing antibody to the prion protein.

The invention further provides a method of determining the nature of prion protein which is present in a sample, comprising the steps of
selecting a reagent capable of improving the binding between prion protein and antibody specifically binding to prion protein,
subjecting the sample to a series of prion protein immunoassays which use the said reagent in various concentrations,
determining the EC₅₀ value of the selected reagent for the prion protein contained in the sample, and
comparing the EC₅₀ value obtained for the prion protein in the sample with the EC₅₀ value of the selected reagent for PrP^{C} and the EC₅₀ value of the selected reagent for PrP^{Sc} to determine the nature of the prion protein contained in the sample.

In some preferred embodiments, the reagent is a metal compound of a prion protein binding metal, in particular a salt or chelate of a metal selected from the group consisting of copper, nickel, zinc and manganese.

In other preferred embodiments, the reagent is an oxidation agent, in particular hydrogen peroxide or a permanganate salt.

The method of the invention is an improved detection method, applicable in various immunoassay formats, including ELISA, affording a signal amplification of some 5 to 10 times or more and resulting in at least a 1000-fold, such as 3000- to 5000-fold enhanced sensitivity of the prion assay. The technique is quick, simple, applicable on serum, plasma, CSF, and urine samples and can be used on brain homogenates or on tissue or cell samples. Furthermore, it is antibody and epitope-independent and can be used with various tags or labels on the antibodies.

The invention will be more fully understood after a consideration of the following description of the invention.

### Brief Description of the Drawings

### Figure 1

Effects of CuSO₄ treatment on PrP^{C} detection in normal human plasma by ELISA. After binding of prion proteins to the microtitre plate (MTP) and washing, the MTP was incubated for 15 minutes with distilled water (open circles) or 400 µM CuSO₄ in distilled water (closed squares). Subsequently the MTP was washed, bound prion proteins were detected with HRP-labeled 1E4 antibody and visualized by TMB substrate. Results are expressed as absorption at 450 nm. Data represent mean absorption ± SD of six determinations. A detailed description of the methodology can be found elsewhere in this patent.

### Figure 2

Effects of various treatments on PrP^{C} detection in normal human plasma by ELISA. After binding of prion proteins to the microtitre plate (MTP) and washing, the MTP was incubated for 15 minutes with distilled water, 50% formic acid, 0.1% H₂O₂, or 400 µM solutions of KMnO₄, CuCl₂, CuSO₄, Cu(NO₃)₂, ZnSO₄, MnCl₂, HgCl₂, MgCl₂, or FeSO₄ , in distilled water. Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 3

Dose-effect relation of Cu(NO₃)₂ treatment on PrP^{C} detection in normal human plasma by ELISA. After binding of prion proteins to the microtitre plate (MTP) and washing, the MTP was incubated for 15 minutes with distilled water (open circles) or various concentrations of Cu(NO₃)₂ in water (other markers). Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 4

Time-effect relation of Cu(NO₃)₂ treatment on PrP^{C} detection in normal human plasma by ELISA. After binding of prion proteins to the microtitre plate (MTP) and washing, the MTP was incubated for various times with 400µM of Cu(NO₃)₂ in distilled water. Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 5

Cu(NO₃)₂ improved detection of PrP^{C} is independent of the epitope of used anti-PrP antibody. After binding of prion proteins to the microtitre plate (MTP) and washing, the MTP was incubated for 15 minutes with distilled water (open markers) or 400 µM Cu(NO₃)₂ in water (closed markers). Subsequently the MTP was washed and bound prion proteins were detected with a series of ¹²⁵I-labeled prion antibodies, directed to various epitopes of the prion protein. Bound radioactivity of the separate wells was determined in a gamma counter. Results are expressed as percentage bound of total counts added. A detailed description of the methodology and used antibodies can be found elsewhere in this patent.

### Figure 6

EDTA-dose related inhibition of the effects of CuCl₂ on PrP^{C} detection in normal human plasma by ELISA. After binding of prion proteins to the microtitre plate (MTP) and washing, the MTP was incubated for 15 minutes with distilled water (AD) or various concentrations of EDTA together with 400 µM of CuCl₂ in water. Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 7

Stimulatory and inhibitory effects of copper and EDTA on the detection of PrP^{C} in normal human plasma. Effects of copper and EDTA are depending on the moment of application within the ELISA. After binding of prion proteins to the microtitre plate (MTP) and washing, the MTP was incubated for 15 minutes with distilled water (AD), copper or EDTA in water. Subsequently the MTP was washed, bound prion proteins were detected with HRP-labeled 1E4 antibody and visualized by TMB substrate. Results are expressed as absorption at 450 nm. At various timepoints in the PrP^{C} ELISA protocol, distilled water (AD), 300 µM CuCl₂ (copper) and/or 3 mM EDTA were added and the effects on the PrP^{C} detection were examined. A detailed description of the methodology can be found elsewhere in this patent.

### Figure 8

Absence of effects of Cu(NO₃)₂ treatment on human IgG (hIgG) detection in normal human plasma by ELISA. After binding of hIgG proteins to the microtitre plate (MTP) and washing, the MTP was incubated for 15 minutes with distilled water (open circles) or 400 µM Cu(NO₃)₂ in distilled water (closed squares). Subsequently the MTP was washed, bound hIgG proteins were detected with HRP-labeled antibody against hIgG antibody and visualized by TMB substrate. A detailed description of the methodology can be found elsewhere in this patent.

### Figure 9

Absence of effects of Cu(NO₃)₂ treatment on human IL-6 (hIL-6) detection in culture supernatant by ELISA. During various steps of the IL-6 assay, as indicated in the legenda, the MTP was incubated for 15 minutes with distilled water (open circles) or 400 µM CuSO₄ in water (closed markers). Subsequently the MTP was washed, and bound hIL-6 was detected with biotin-labeled hIL-6 antibody. Bound complexes were visualized by streptavidine-poly-HRP incubation and TMB substrate. Results are expressed as absorption at 450 nm. A detailed description of the methodology can be found elsewhere in this patent.

### Figure 10

Overview of specific effects of Cu(NO₃)₂ treatment on ELISA detection of natural prion proteins in various samples and its absence on recombinant prion proteins and several other human plasma proteins.

After binding of proteins to the microtitre plate (MTP) and washing, the MTP was incubated for 15 minutes with distilled water (white bars) or 400 µM Cu(NO₃)₂ in water (black bars). Subsequently the MTP was washed, bound proteins were detected directly (HRP labeled antibody) or indirectly (biotin labeled antibody followed by HRP-labeled streptavidin) and visualized by TMB substrate. Results are expressed as absorption at 450 nm. A detailed description of the methodology can be found elsewhere in this patent.

### Figure 11

Effects of Proteinase K (PK) treatment on copper-induced assay sensitivity improvement of prion detection in hamster 263K scrapie infected brain homogenate. Brain homogenates of scrapie-infected hamster were incubated for 30 minutes at 50 °C without (continuous lines) or with PK (dotted lines) and denaturated. After binding of proteins to a nitrocellulose coated microtitre plate (NC-MTP) and washing, the NC-MTP was incubated for 15 minutes with distilled water (open markers) or 400 µM Cu(NO₃)₂ in water (closed markers).

Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 12

Dose-effect relations of CuCl₂ , ZnCl₂ and MnCl₂ treatment on PrP^{C} detection in normal human plasma by ELISA. After binding of prion proteins to the microtitre plate (MTP) and washing, the MTP was incubated for 15 minutes with various concentrations of CuCl₂ (circles), ZnCl₂ (triangles) or MnCl₂ (squares) in water. Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Figure 13

Direct effects of EDTA or CuCl₂ on the migration of plasma PrP^{C} in an ultracentrifuge sucrose gradient. Normal human plasma was mixed with EDTA (10 mM final concentration) or CuCl₂ (400 µM) and separated onto a 5-30% sucrose gradient, run for 16 hours at 100,000 x g. Fractions of various sucrose densities were bound to the microtitre plate (MTP). Subsequently the MTP was washed, bound prion proteins were detected with HRP-labeled 1E4 antibody and visualized by TMB substrate. Results are expressed as absorption at 450 nm. A detailed description of the methodology can be found elsewhere in this patent.

### Detailed Description of the Invention

The invention provides an immunoassay method for detecting or measuring prion protein in a sample using at least one antibody specifically binding to prion protein, comprising contacting the prion protein present with a reagent capable of improving binding of said at least one antibody to the prion protein.

Herein, the term "immunoassay method" has a broad meaning, covering not only immunoassays applied to a liquid material, such as a body fluid (for example plasma, serum, urine, CSF, and others) or a tissue homogenate (for example brain homogenate), but also immunohistochemical or immunocytochemical methods, including for example cytospin methods (involving separation of cells from a source material, for example by centrifugation, contacting the thus separated cells with a labelled detection antibody specifically binding to prion protein, and examining the binding of said detection antibody to the cells).

As a result, the "sample" in the method of the invention can be liquid or solid, it can be a body fluid, such as plasma, serum, CSF, urine, and any other liquid that may contain prion protein, including tissue homogenate, such as brain homogenate, or can be a tissue or cell sample, such as thin slices of brain tissue and cells separated by cytospinning.

As used herein, "prion protein" refers both to normal prion protein and to aberrant (pathological) prion protein, of any species, especially mammals, including human. The invention is especially applicable and effective with natural (or native) prion protein, as contrasted to recombinant prion protein. Natural prion protein is prion protein as it is found in and isolated from a mammal. The invention works very well with any normal natural prion protein.

In one group of preferred embodiments, said reagent is a metal compound of a prion protein binding metal. It is known that metal ions of copper, nickel, zinc and manganese bind to prion protein. In view thereof, it is preferred that said metal compound is a salt or chelate of a metal selected from the group consisting of copper, nickel, zinc and manganese. Specific examples of suitable metal salts are CuCl₂, CuSO₄, Cu(NO₃)₂, ZnSO₄, ZnCl₂, Zn(NO₃)₂, NiCl₂, NiSO₄, Ni(NO₃)₂, MnCl₂, MnSO₄ and Mn(NO₃)₂. Chelates of the above mentioned metals are suitable as well, in particular chelates formed with an amino acid. As an example thereof, copper glycine chelate can be mentioned. Salts of copper, zinc and manganese are preferred, especially salts of copper and zinc.

Most preferably, the metal compound is a copper salt. It appears that copper salts are more effective than salts of other metals.

In another group of preferred embodiments, said reagent is an oxidation agent. Preferably, said oxidation agent is hydrogen peroxide or a permanganate salt, such as sodium or potassium permanganate.

Most preferably, the oxidation agent is a permanganate salt. It appears that permanganates are more effective than other oxidation agents, such as hydrogen peroxide.

Copper, nickel, manganese and zinc salts are used preferably in a range from 10 nanomole up to 3 mole per liter, more preferably for copper salts in a range from 100 nanomole up to 100 micromole per liter, while zinc and manganese salts are more preferably used in a range from 1 micromole up to 100 millimole per liter. KMnO₄ and H₂O₂ are active in the range of 10 micromole to 3 moles per liter, more preferably used in a range from 100 micromole up to 300 millimole per liter.

In the immunoassay method, at least one antibody specifically binding to prion protein is used. Said antibody may be a catcher antibody used for immobilizing the prion protein to a solid surface, or a detection antibody used for detecting the prion protein immobilized onto the solid surface, or both. The present invention improves the binding between prion protein and antibody specifically binding to prion protein, irrespective of the function of the antibody in the assay.

Although the present invention is broadly applicable, as observed above, to any immunoassay methodology compatible with the nature of prion protein, and covers immunohistochemical and immunocytochemical methods, a highly desirable immunoassay method applies to the determination of prion protein in a liquid sample, such as a body fluid or tissue homogenate, and comprises contacting the sample with a solid surface to immobilize prion protein present in the sample to said solid surface, contacting the solid surface, optionally after washing to remove components of the sample which have not been bound to the solid surface, with the reagent, contacting the solid surface, optionally after washing to remove reagent which has not been bound to the solid surface, with a detection antibody specifically binding to prion protein, and determining, optionally after washing to remove detection antibody which has not been bound to the solid surface, the presence or amount of detection antibody which has been bound to the solid surface. Although each of the above mentioned washing steps is facultative, it is preferred that all of these washing steps are included.

Although any immunoassay format may be used, including competitive and non-competititve, using any kind of labeling substance, including radioactive labels, enzyme labels, luminescent labels, dye labels, the immunoassay preferably is in ELISA format.

In one embodiment of such ELISA, the detection antibody used carries a detectable enzyme label and the presence or amount of detection antibody bound to the solid surface is determined by detecting or measuring the conversion of a substrate of said enzyme.

In another embodiment of such ELISA, the solid surface, after said contacting with the detection antibody, is contacted with an antibody specifically binding to the detection antibody and carrying a detectable enzyme label and wherein the presence or amount of detection antibody bound to the solid surface is determined by detecting or measuring the conversion of a measurable substrate of said enzyme.

In a preferred embodiment of the immunoassay method the prion protein present in the sample is bound by adsorption directly to the solid surface. Prion protein is a very sticky substance and can be bound easily to a solid phase without using a capturing antibody for that purpose. However, the invention includes immunoassay methods which make use of a capturing antibody or other material for capturing the prion protein and binding it to the solid surface. Some peptides or proteins have high affinity for prion protein (normal prion protein, pathogenic prion protein, or both), for example fibrinogen, plasminogen, clusterine and many others, and they are all useful for immobilisation of the prion protein to the solid surface. The nature of the solid surface may be specifically adapted to the binding of prion protein.

The solid surface may be pretreated, or be of a nature, such that the binding of other substances than prion protein is prevented or reduced. The nature of sample dilution buffer may be specifically adapted to improve the binding of prion proteins and reduce the binding of other substances than prion proteins.

This invention also provides a method of separating prion protein from a prion protein containing solution using a capturing antibody specifically binding to prion protein, comprising contacting the prion protein present with a reagent capable of improving binding of the capturing antibody to the prion protein.

As to the reagent capable of improving binding of the capturing antibody to the prion protein, the same materials as described above for the immunoassay method can be used.

In a practical method, the prion protein containing solution after being treated with the reagent is passed through a filter or affinity chromatography column containing capturing antibody immobilized to a solid carrier and the prion protein depleted solution, or the separated prion protein, or both, are collected. Thus, the invention improves existing methods for collecting prion protein, and existing methods for depleting prion protein from solutions containing the same, such as blood.

The present invention is based on the realisation that the immunological detection of natural prion proteins in various biological matrices is suboptimal and that it can be significantly improved by a specific treatment as described herein. When prion proteins, bound to the microtitre plate, are treated with an oxidizing agent or a Cu, Mn, or Zn salt containing agent, a dose-dependent and incubation-time dependent improvement of detection by prion antibodies was found. Various agents were tested, including H₂O₂, KMnO₄, CuCl₂, CuSO₄, Cu(NO₃)₂, ZnSO₄, MnCl₂, and they all showed an improvement in sensitivity for the detection of prion proteins. When tested with 4 different antibodies against epitopes distributed over the prion protein, the resulting immunoassay turned out to have an increased sensitivity in all cases. The assay improving effects of copper were specific for prion assays and could be blocked dose dependently by the addition of EDTA.

We hypothesize, without being bound to this explanation, that a treatment with the above mentioned salts or oxidative agents has an effect on the threedimensional (3D) structure of the prion protein, which potentially can have considerable impact in various fields of prion research, human and veterinary TSE diagnostics, the safety of blood and blood derived products, and food industry. The antibody binding improving effect of a treatment according to this invention may be that accessibility of the prion protein is increased, for example as a result of a conformation change, or as a result of breaking up conglomerates or complexes of the prion protein with itself or with other substances.

Since changes in conformation are essential in the detection of PrP^{Sc}, thus on PK resistance, solubility and aggregation of the prion protein, this invention can help to restore the 3D structure, making protease discrimination between PrP^{C} and PrP^{Sc} in plasma or serum samples possible. Also complexes of prions and other proteins in blood samples are 3D dependent, and influencing the conformational structure of prion proteins will have its effect on availability of free, nonaggregated and unbound prion proteins.

The surprising finding that binding of antibodies to prion proteins is considerably improved after metal salt or oxidative treatment, is a feature that can be used in affinity chromatography and/or filter applications for removal of prions from biological samples, reducing the possible risk of TSE transmission.

Also in diagnostic applications this invention will increase the sensitivity of the assay and can be applied at various moments of the prion assay. Metal salts or oxidative treatments can be used to improve the binding onto a variety of prion capturing surfaces, including specific matrices, peptides, proteins, receptors, monoclonal or polyclonal antibodies, phage antibodies or fragments thereof, directly or indirectly bound via chemical linkers, photochemical linkers, biotin or (strept)avidin. The solid surfaces include microtitre plates, beads, pins, sticks or membranes, but not in all situations binding onto a surface is necessary in the first step of the assay. In so-called liquid face interactions binding onto a solid face is completed in a later stage. Binding of prions onto these capturing surfaces can be completed in a variety of buffers with a pH in the range from 3-10, possibly containing additives to prevent nonspecific binding of other proteins, including blocking proteins, peptides, salts, surfactants, polyethylene glycols and the like.

The invention can also be used after binding onto a certain specific catcher, thus improving the detection of bound prion proteins in various assays. Detection of bound prion proteins can be completed on a variety of prion capturing surfaces, including specific matrices, peptides, proteins, receptors, monoclonal or polyclonal antibodies, phage antibodies or fragments thereof. In case of an assay with liquid face interaction previously mentioned, these surfaces include microtitre plates, beads, pins, sticks or membranes. Washing steps between binding and detection of prions can be accomplished, however are not necessary in all types of assays.

The subject invention can also be used in immunohistochemical applications. Microscopical detection of prion proteins in tissue samples, e.g. sections of brain, spleen, tonsils, or appendix, is often cumbersome due to the low local concentrations. We believe that this invention can give a more intensified signal, facilitating clinical and post-mortem diagnosis of prion diseases and the like.

Visualisation of captured prion proteins can be obtained via a wealth of detection techniques, including radioactivity, enzyme activity, fluorescence, chemiluminescence, detection of particles, (electron) microscopy, solid phase resonance, refractometer and other light scattering techniques.

Another possible application of this invention is to detect the metal-binding capacities of prion proteins and its possible aberrant behaviour in samples from diseased human and animals. Determination of EC₅₀ values, e.g. determination of the concentration of metal salt that will give 50% of the maximal outcome in the detecting assay, can be a very efficient approach. Comparison of titration curves obtained EC₅₀ values of various metal salt can give information on the presence of PrP^{C} or PrP^{Sc} in the biological sample and discriminate between healthy and diseased human or animal.

Thus, the invention further provides a method of determining the nature of prion protein which is present in a sample, comprising the steps of
selecting a reagent capable of improving the binding between prion protein and antibody specifically binding to prion protein,
subjecting the sample to a series of prion protein immunoassays which use the said reagent in various concentrations,
determining the EC₅₀ value of the selected reagent for the prion protein contained in the sample, and
comparing the EC₅₀ value obtained for the prion protein in the sample with the EC₅₀ value of the selected reagent for PrP^{C} and the EC₅₀ value of the selected reagent for PrP^{Sc} to determine the nature of the prion protein contained in the sample.

The invention will be illustrated with some non-limiting examples below. They show that treatment of a series of biological samples, including serum, EDTA plasma, citrate plasma, heparin plasma, platelet concentrates, CSF and brain homogenates with a copper salt or some other materials resulted in an improved, more sensitive detection of PrP^{C} in prion assays. Whereas in non-prion assays, independently of the assay format, no improvement on the assay sensitivity can be found after oxidation.

### Examples & Methodology

### Example 1: Effects of CuSO₄ on prion detection

Prion proteins have the ability to bind copper. However, in EDTA plasma samples all available Cu⁺⁺ will be chelated by EDTA. The antibody in the prion assay used, MAb 1E4, has its epitope in the human PrP₁₀₀₋₁₁₁ amino acids (AA) sequence, which sequence is a few amino acids away from the Cu-binding octarepeats.

It can be hypothesized that in the presence of Cu-ions the prion protein due to a different charge may have another conformation than in the absence of Cu-ions. Hence, we tested whether Cu⁺⁺-ions influenced the detection of prion protein in the assay.

A pool of normal human plasma from at least 20 healthy volunteers, containing 10 mM sodium EDTA, was serially diluted in Dilution Buffer (DB= 20 mM Tris/HCl, pH 7.5, 0.02 % Tween-20). Forty microlitres of each dilution step was transfered to a well of a 96-well microtitre plate (NUNC) and incubated for 1 h at room temperature. After washing 5 times with washing buffer (PBS + 0.02% Tween-20), wells were incubated for 15 min with 40 µl distilled water (AD) or 40 µl CuSO₄ (400 µM in AD). Subsequently the wells were washed, incubated with HRP-labeled prion MAb 1E4 for 1 h and washed again. Bound antibody was then visualized by TMB substrate. For better reproducibility and maximal assay performance all incubation steps were performed on an orbital shaker (700 ± 100 rpm).

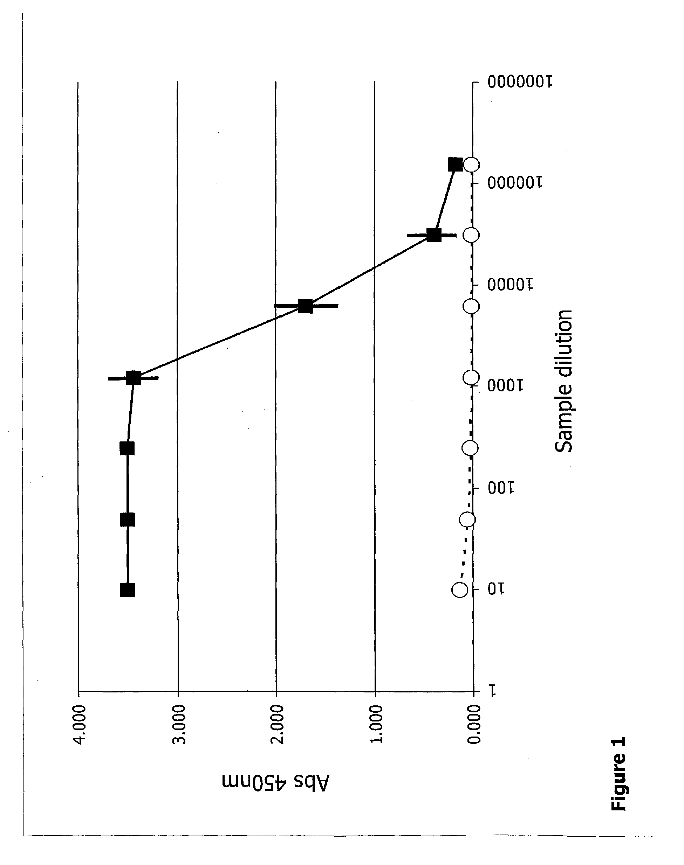

As shown in figure 1, treatment of bound prion proteins from human plasma with CuSO₄, resulted in a spectacular increase in sensitivity of the assay, twice the backgound signal was detected at 50 and 156250 times diluted plasma of AD and CuSO₄ treated plasma, respectively, i.e. a more than 3000-fold increase in sensitivity.

### Example 2: Other amplificating examples

CuSO₄ has oxidative as well as acidic chemical properties. Therefore, we tested other oxidative as well as acidic substances for their effects in the assay, using the above mentioned protocol. From each chemical compound its chemical property and maximal effective concentration is mentioned between brackets: H₂O₂ (0.1 % , oxidative), formic acid (50 %, acidic), KMnO₄ (400 µM, oxidative), CuCl₂ (400 µM, oxidative), CuSO₄, Cu(NO₃)₂, ZnSO₄, (all 400 µM, acidic & oxidative), MnCl₂, HgCl₂, MgCl₂ (400 µM, oxidative) or FeSO₄ (400 µM, acidic & oxidative).

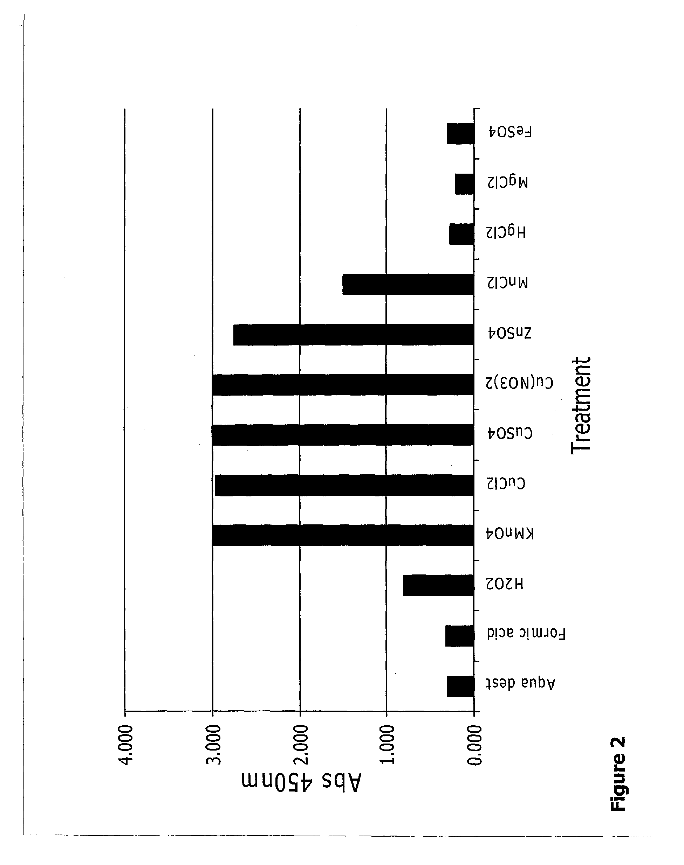

As shown in figure 2, the oxidative treatments (H₂O₂, KMnO₄ )enhanced the detection of bound prion proteins, while the acidic (formic acid) treatment had no effect on the assay. Furthermore when we tested the metal-salt solutions, only the Cu-, Mn- and Zn- salts had positive effects on the assay, while Hg, Mg and Fe salts had no effects on the prion assay sensitivity. This is in accordance with results showing that especially these metals bind to PrP^{C} with high affinities [12], therefore we can conclude that for the metal dependent increase in sensitivity, binding to the prion protein is necessary.

### Example 3: Dose- and time-dependent effects of various agents

As shown in the previous paragraph, many oxidizing compounds and metal salts are able to improve the sensitivity of the prion assay. In a next experiment especially the dose-reponse and the time-kinetics of Cu containing salt on assay sensitivity was investigated. Following the above mentioned protocol, various concentrations of Cu(NO₃)₂ in AD were tested with an incubation time of 15 min.

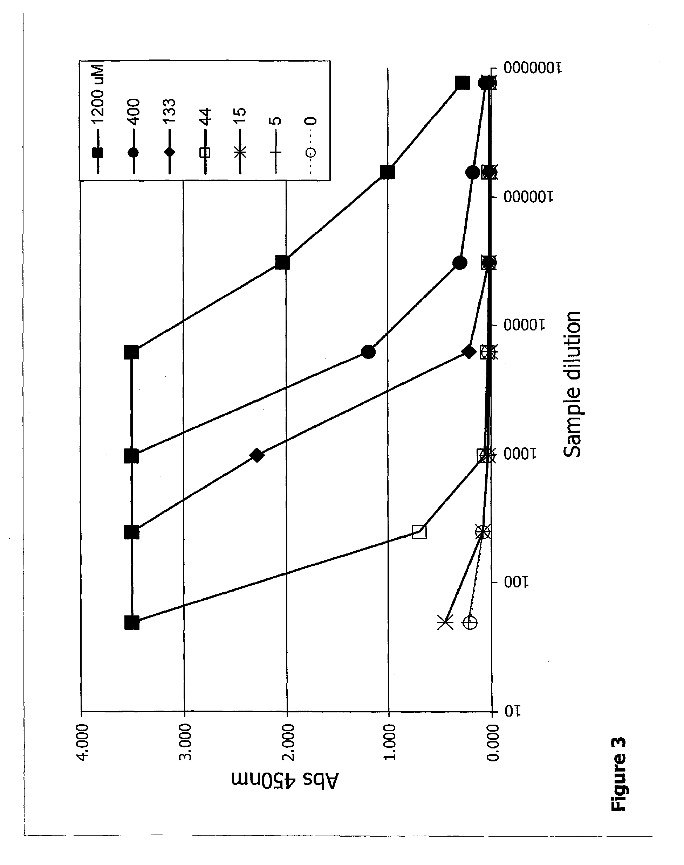

As shown in figure 3 a clear dose related effect was observed. Fifteen µM was the minimal concentration of Cu(NO₃)₂ that showed the enhancing effect on the assay. At the highest dose 1200 µM, also the background signal slightly elevated, therefore a concentration of 400 µM was chosen for further experiments. Some of the other compounds mentioned above (H₂O₂, KMnO₄, CuSO₄) were tested and also showed dose-related effects on the assay sensitivity. Cu(NO₃)₂ and CuCl₂ were chosen for further experiments because the use of these compounds yielded in minimal variation in the assay.

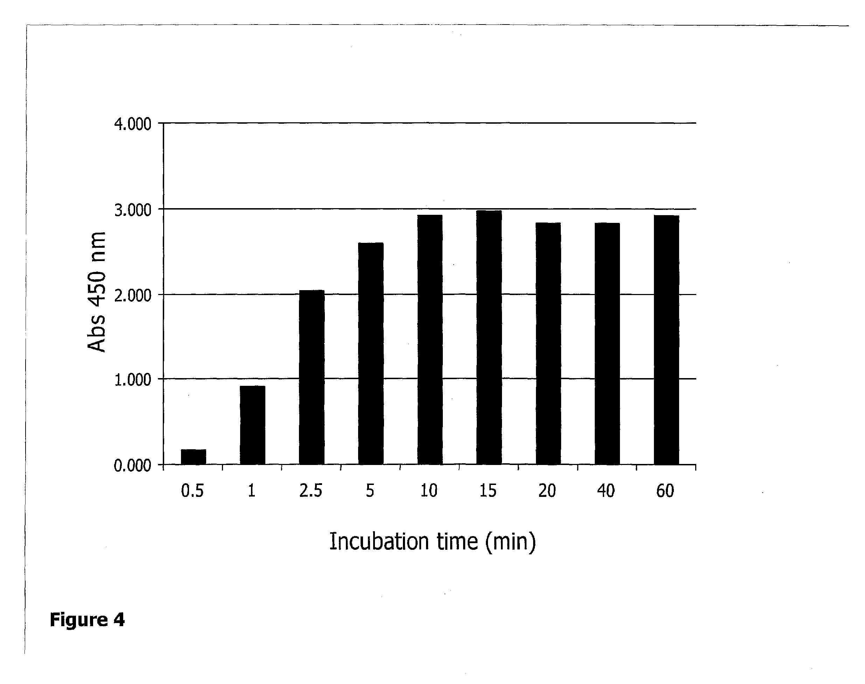

In figure 4 it is demonstrated that the amplifying effect of 400 µM Cu(NO₃)₂ on the prion assay is definitely time-dependent, being optimal after 10-15 minutes.

### Example 4: Are copper effects on prion detection epitope-dependent?

When using phage antibodies, Williamson described three different linear epitope regions within the prion protein [30]. Especially epitope I would be "hidden" within the natural prion protein to become exposed only after denaturation. To evaluate whether the effect of copper on prion proteins was limited to a certain part of the molecule, or whether it was relevant for the whole protein, 4 mAbs against different epitopes, mAb FH11 (epitope located on the N-terminal part), mAb BE12 (epitope located around AA 93-99), mAb AH6 (AA 159-175), all obtained from the TSE Resource Centre, Institute for Animal Health, Compton, Newbury, Berkshire, UK), as well as mAb 1E4 (AA 100-111) were tested. To rule out effects of possible oxidation in the the previous incubations on the TMB conversion in the final step (which is oxidation dependent), ¹²⁵I-labeled antibodies were used allowing direct assessment of binding of mAbs to the prion protein.

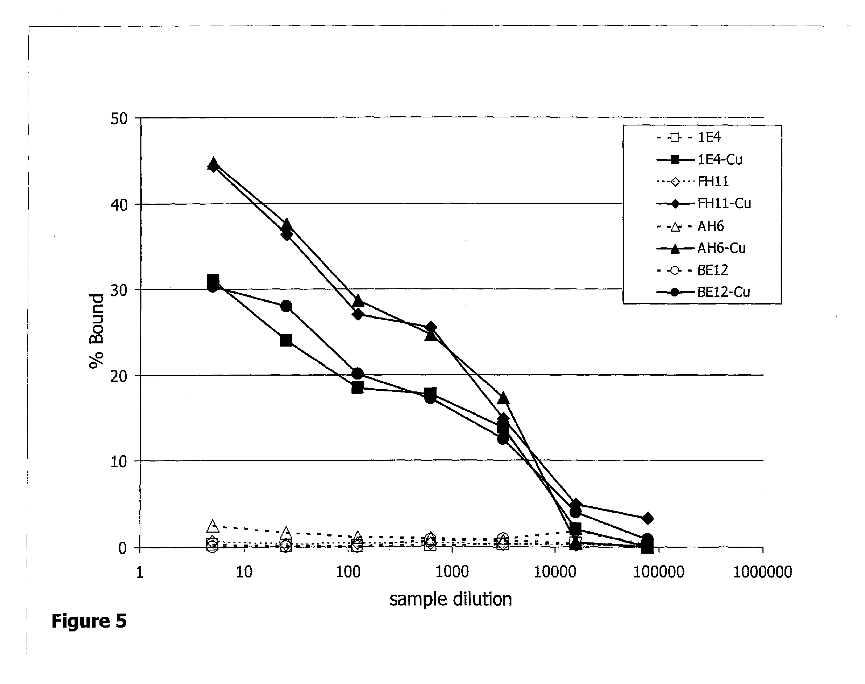

As shown in figure 5, all four mAbs, directed against different epitopes of the prion protein, showed enhanced binding upon 15 minutes treatment with 400 µM Cu(NO₃)₂, indicating that this phenomenon is not restricted to certain parts of the prion protein. Thus, the observed effect was distinct from epitope hiding as described by Willamson [30].

### Example 5: Blocking the copper effects on prion detection

In another approach to ensure that the enhancing effects on PrP^{C} detection are copper-specific, the above mentioned protocol was performed while Na₂EDTA at various concentrations was added in the copper incubation step. Instead of Cu(NO₃)₂ we used CuCl₂ (400 µM in AD) to avoid acidic effects of the copper containing agent.

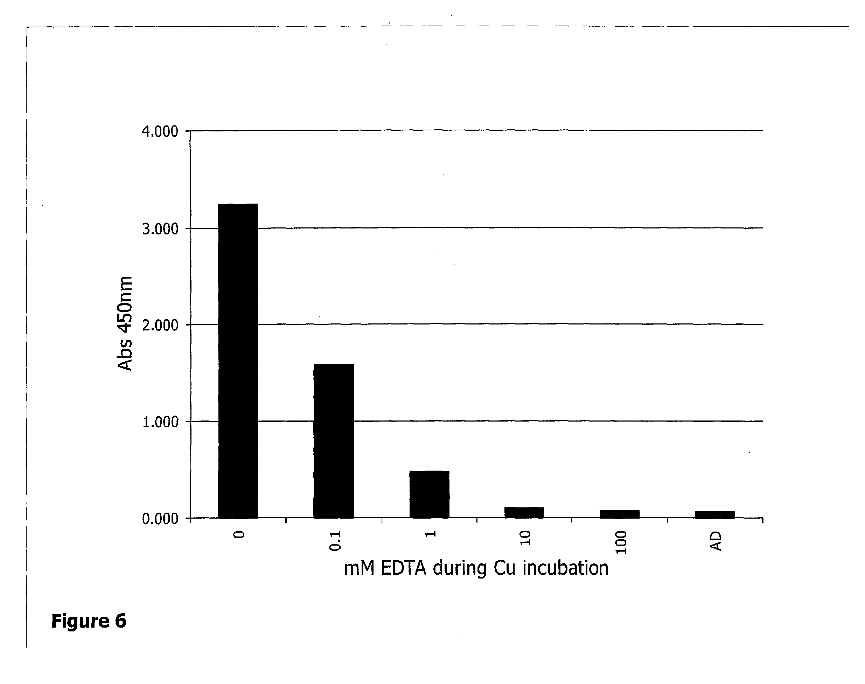

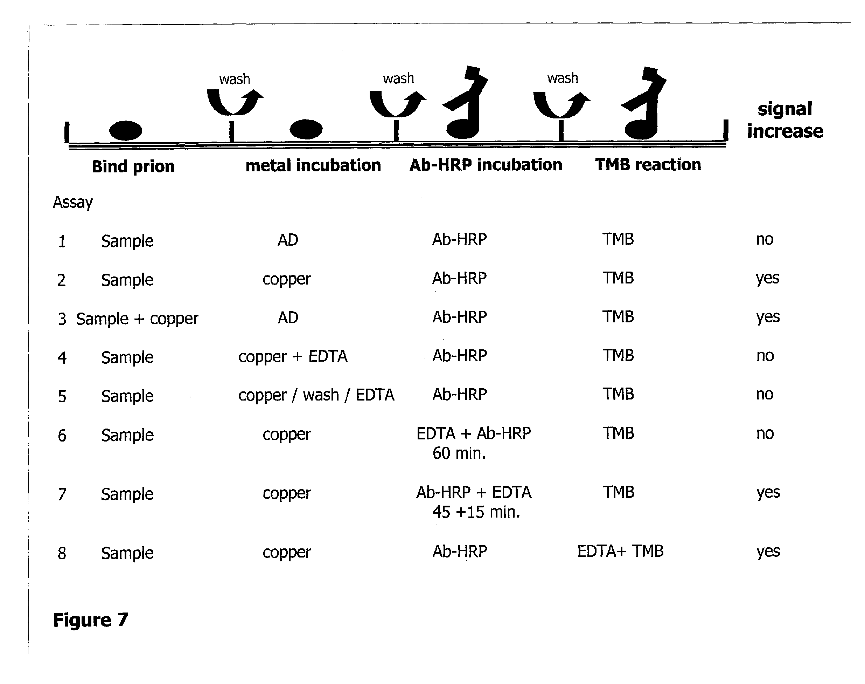

A clear dose-related reduction of copper-induced assay sensitivity increase was found, as shown in figure 6, indicating that chelating free Cu⁺⁺ ions by EDTA blocks the assay improving effects, another proof of specific copper-driven effects on the assay.

### Example 6: Stimulatory and inhibitory effects of copper and EDTA on the detection of PrP^{C} in normal human plasma

Blockade of the copper effect in the ELISA with EDTA described in example 5 was performed only in the copper incubation step of the PrP^{C} ELISA. Using 1000-fold diluted human plasma, distilled water (AD), 300 µM CuCl₂ (copper) and/or 3 mM EDTA were added in various steps, and the effects on the PrP^{C} detection were examined. Assay 1 is the standard situation, without copper addition, resulting in a weak detection of PrP^{C} as mentioned in example 1. Assay 2 descibes the results with copper added after prion binding to the MTP, resulting in a much more improved detection of PrP^{C} as described in example 1. In assay 3 CuCl₂ was mixed with the sample and together incubated in the MTP. This resulted in a slightly worse detection of PrP^{C} as compared to assay 2. This somewhat less detection of PrP^{C} in assay 3 was possibly due to the binding of copper to other proteins present in the plasma sample, resulting in a less efficient binding to PrP^{C}, or to less efficient binding of PrP^{C} to the MTP due to the presence of copper. Yet, the sensitivity of detection of PrP^{C} in assay 3 was much greater than that of assay 1. Assay 4 shows the blocking effects of EDTA on copper-induced effects when simultaneously added to the copper. Simultaneous addition of EDTA to copper reduces availability of free copper, and hence prevents the effect of copper on PrP^{C}. In assay 5 bound PrP^{C} was first saturated with copper, like in assay 2, however after incubation with copper the MTP was washed, and incubated with EDTA for 15 minutes. This resulted in a complete inhibition of the copper effects, indicating that EDTA completely reverses copper-induced changes in PrP^{C}. Assays 6 and 7 are almost identical protocols, however when EDTA and HRP-labeled antibody 1E4 are brought in contact with copper-loaded PrP^{C} at the same time (assay 6), the copper-induced changes of PrP^{C} disappeared. This is in contrast with the situation in assay 7 in which mAb 1E4 is allowed to bind to PrP^{C} for 45 minutes, followed by addition of EDTA for the last 15 minutes. In the latter assay improved detection of PrP^{C} was observed, indicating that mAb 1E4 had stabilized copper-induced conformation of PrP^{C}.

In conclusion, the addition of copper changes the 3D-structure of the prion protein dramatically, resulting in a more efficient binding of 1E4 and other monoclonal antibodies, independent of the antibody epitope. Furthermore this phenomenon can be reversed by incubation with EDTA, as long as copper-induced conformation is not stabilized by antibody binding.

### Example 7: Specificity of assay amplification

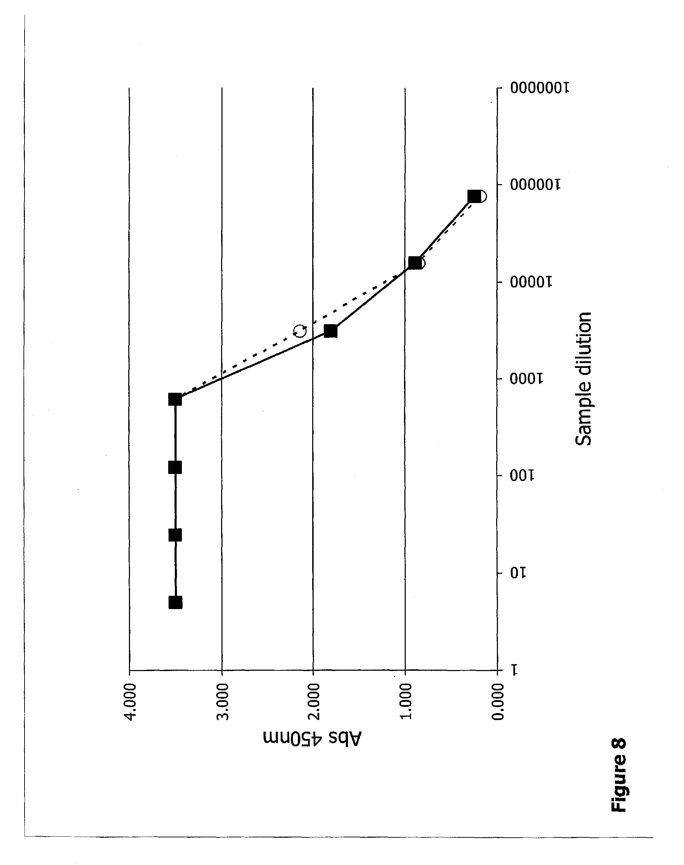

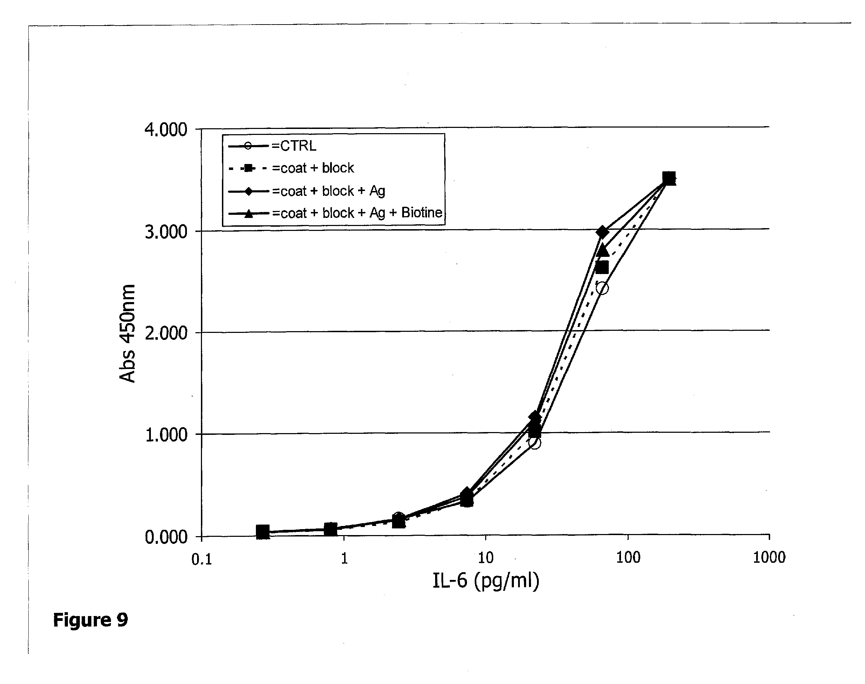

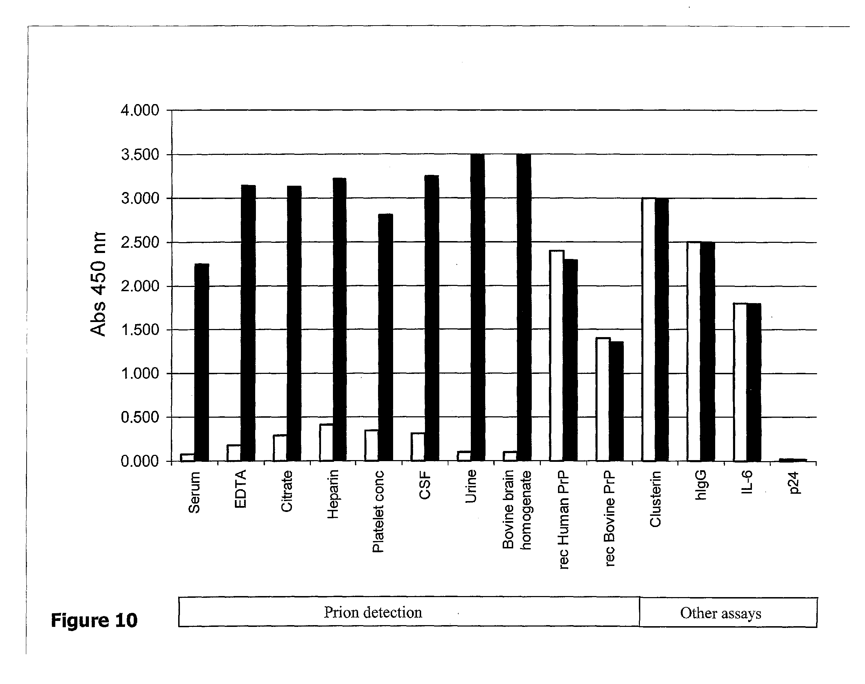

One may postulate that the observed effect of copper on the binding of antibodies in the prion assay, is not specific for prion proteins, but is a general phenomenon of immunoassays. Therefore, the effect of Cu(NO₃)₂ was tested in various immunoassays with normal human poolplasma. Different protocols were evaluated: detection of human IgG (figure 8), with a directly labeled mAb to human IgG, thus in an identical setting as the prion assay described earlier in the document. Also the effects of copper in an indirect ELISA with biotin labeled antibodies (IL-6 ELISA, figure 9) and HRP-labeled streptavidin were studied for clusterin (Figure 10) and HIV-related p24 (Figure 10). The latter assay was included as it should remain negative when normal pool plasma was tested. Finally the effects of copper at different steps in a sandwich IL-6 ELISA were also studied (figure 9).

The results clearly showed that the effect of Cu(NO₃)₂ was exclusive for natural prion proteins, no effect was found on recombinant prion proteins and it was was not a general feature of immunoassays.

Conclusively we can state that the copper treatment of a series of biological samples, including serum, EDTA plasma, citrate plasma, heparin plasma, platelet concentrates, CSF and brain homogenates all resulted in at least a ten-fold increase in signal in TMB colour formation and associated improved sensitivity of the prion assays. Whereas in non-prion assays, independently of the assay format, no improvement on the assay sensitivity can be found after oxidation.

### Example 8: Effects of Proteinase K digestion on assay amplification

The octa-repeats, present in the N-terminal domain of PrP, are believed to be involved in the copper-binding properties of prions. Treatment of brain homogenates with proteinase K (PK) results in a complete digestion of PrP^{C} while PrP^{Sc} is relatively resistant for digestion and only the first 90 amino-acids of the N-terminal domain are removed, which is exactly the octa-repeats containing part of the prion protein.

Therefore we investigated the effects of PK digestion on the copper-induced enhanced assay sensitivity using a homogenate from a 263K scrapie infected hamster brain. We performed a direct ELISA using a nitrocellulose-coated microtitre plate, which has a high affinity for binding PrP^{Sc}. Nitrocellulose was dissolved in ethanol to a final concentration of 1 mg/ml. A volume of 40 µl of this mixture was added into each well of a 96-well microtitre plate (NUNC maxisorp type F96) and the solvent was evaporated by heating the plate in an oven for 30 minutes at 80°C resulting in a clear nitrocellulose layer remaining at the bottom of the microtitre plate. Brain homogenates of scrapie-infected hamster (10 % in PBS) were further diluted to a final concentration of 0.1 % with a digestion buffer (100mM Tris/HCl pH 7.5, 0.05% SDS) and incubated for 30 minutes at 50 °C with or without PK (final concentration: 30 µg/ml ~ 0.6 U/ml). After treatment the volume of the mixture had increased by one-third due to addition of sample buffer (200 mM TRIS/HCl [pH 8.5], 6% SDS). The mixture was incubated for 10 min at 96°C. Subsequently 40 µl of treated sample was transferred to the wells of the nitrocellulose-coated microtitre plate and incubated for 1 hour at room temperature (RT) while shaken. After washing with washing buffer (PBS, 0.02% Tween-20), following the above mentioned protocol the assay was completed.

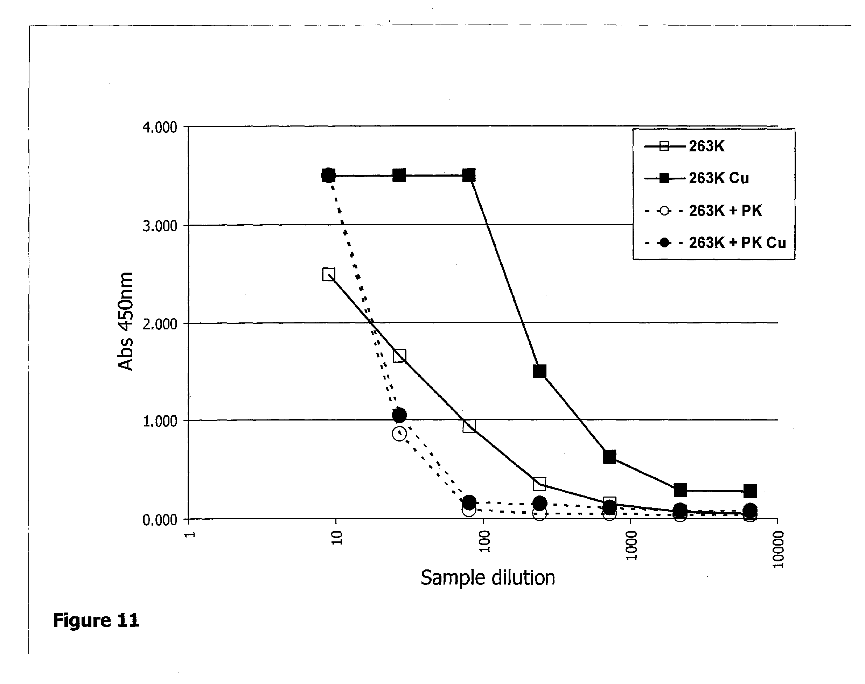

As shown in Figure 11, copper treatment only was effective in non-digested brain homogenates. Since non-digested 263K brain homogenate contains as well cellular (PrP^{C}) as scrapie (PrP^{Sc}) proteins and it remains unclair at this moment to what account both conformations contribute to the signal improvement after copper treatment. Digested 263K brain however, only contains PrP^{Sc} and are missing the octarepeats due to the PK digestion, which results, as shown in figure 11, in a complete loss of copper efficacy on the assay sensitivity.

### Example 9: Comparison of dose-dependent effects of copper, zinc and manganese

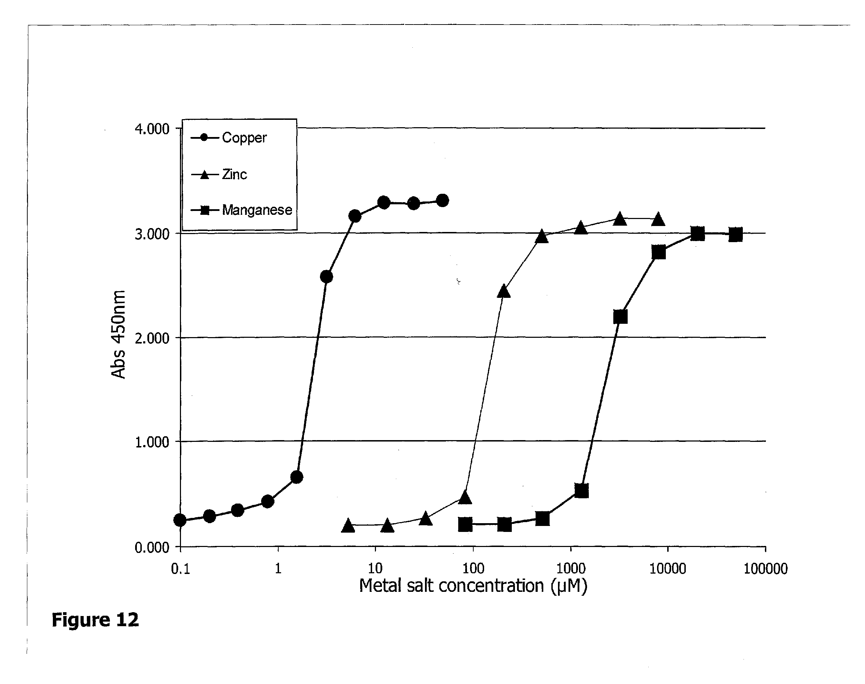

As shown in the previous paragraphs, many oxidizing compounds and metal salts are able to improve the sensitivity of the prion assay. In this experiment the relationship between metal salts was studied by comparison of the dose-related effects on the assay results. Following the above mentioned protocol, EDTA plasma was diluted thousandfold in PBS and serial dilution of various concentrations of CuCl₂, ZnCl₂ or MnCl₂ in water were tested with an incubation time of 15 minutes.
As shown in figure 12, differences in dose-related effects were observed for each metal salt tested. Comparison of the EC50 values, e.g. comparison of the specific concentration of metal salt that will give 50 % of the maximal signal in the assay, can give information on the relative effects of different metal salts with prions present in the sample. In this case the EC₅₀ ratios of Copper : Zinc : Manganese is 1 : 80 : 1000 , this means that about 1000 times higher concentrations of manganese are necessary to obtain the same signal increasing effect as compared to copper and about 12 times more as compared to zinc. Since it is known that conformational changes can have effect on the metal binding properties of prion proteins, we expect that the dose-related effects of various metals in prion assays in diseased human or animals may differ from healthy humans or animals. Using equilibrium dialysis, Brown and collegues [2] described a copper saturation plot for recombinant human PrP₂₃₋₉₈ with an effective copper concentration range almost idential to the range observed in figure 12.

### Example 10: Effects of EDTA or copper on the migration pattern of plasma PrP^{C} in an ultracentrifuge sucrose gradient sedimentation run

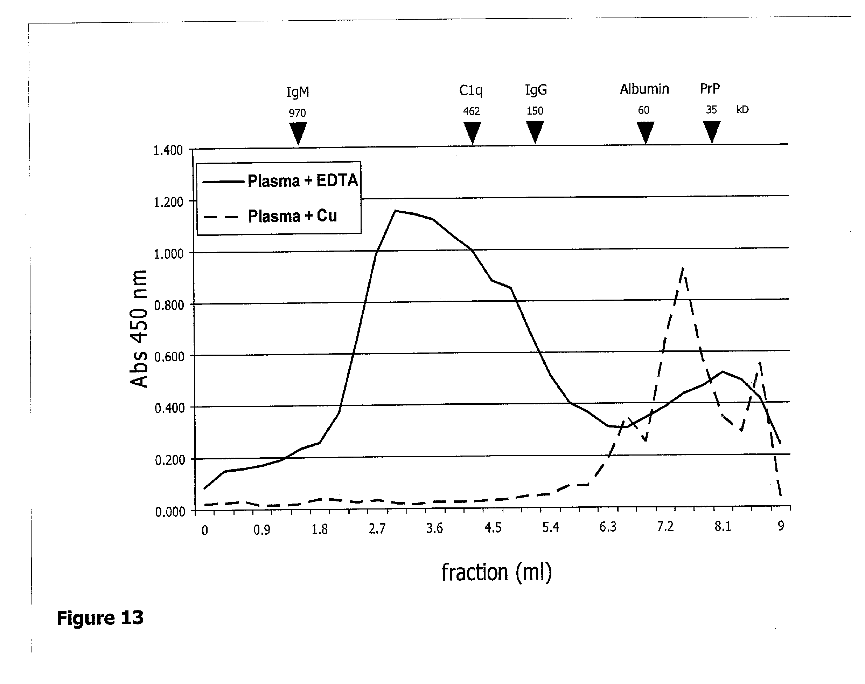

To investigate the effects of EDTA or copper on PrP^{C} folding and possible changes in aggregation, normal EDTA plasma was mixed with EDTA (final concentration of 10 mM) or CuCl₂ (400 µM). Subsequently 500 µl of copper-plasma or EDTA-plasma was loaded on top of a 12 ml tube, containing a 5-30% sucrose gradient in 10 mM EDTA or in 400 µM CuCl₂ in destilled water. After centrifugation for 16 h at 100,000 x g and at 4 °C, 300 µl fractions were collected, diluted five times in PBS and bound to the microtitre plate (MTP), and tested according to the above mentioned protocol, but without the copper incubation step later in the prion assay. As shown in figure 13, plasma EDTA showed two distinct peaks of PrP^{C} immunoreactivity, one small peak close to the molecular weight of monomeric PrP (35 kD), and a large peak with a molecular weight of approximately 650 kD. Whether these high molecular weight complexes consist of polymeric PrP protein only (18-20 PrP proteins together) or macromolecular complexes of PrP with other proteins is still under investigation. When plasma was mixed with copper and fractionated on a copper containing sucrose gradient, the high molecular weight peak, present in EDTA plasma, disappeared and only one peak, close to the molecular weight of monomeric PrP, remained and increased in PrP immunoreactivity. We conclude that under normal conditions a large part of the prion proteins are present in aggregates and that after metal treatments like copper, zinc or manganese, these aggregates dissociate and epitopes on prions become more accessible for detection by specific antibodies.

### Reference List

1 Brown, D. R. and Mohn, C. M. (1999) Glia 25, 282-292
2 Brown, D. R., Qin, K., Herms, J. W., Madlung, A., Manson, J., Strome, R., Fraser, P. E., Kruck, T., von Bohlen, A., Schulz-Schaeffer, W., Giese, A., Westaway, D., and Kretzschmar, H. (1997) Nature **390**, 684-687
3 Brown, P., Rohwer, R. G., Dunstan, B. C., MacAuley, C., Gajdusek, D. C., and Drohan, W. N. (1998) Transfusion **38**, 810-816
4 Chan, S., Gerson, B., and Subramaniam, S. (1998) Clin.Lab Med. **18**, 673-685
5 Collinge, J., Rossor, M. N., Thomas, D., Frosh, A., and Tolley, N. (1998) BMJ **317**, 472-473
6 Gennis, R. B. (1998) Science **280**, 1712-1713
7 Harris, Z. L. and Gitlin, J. D. (1996) Am.J.Clin.Nutr. **63**, 836S-841S
8 Hornshaw, M. P., McDermott, J. R., and Candy, J. M. (1995) Biochem.Biophys.Res.Commun. **207**, 621-629
9 Hornshaw, M. P., McDermott, J. R., Candy, J. M., and Lakey, J. H. (1995) Biochem.Biophys.Res.Commun. **214,** 993-999
10 Houston, E. F. and Gravenor, M. B. (2003) Vet.Rec. **152**, 333-334
11. Houston, F., Foster, J. D., Chong, A., Hunter, N., and Bostock, C. J. (2000) Lancet **356**, 999-1000
12 Jackson, G. S., Murray, I., Hosszu, L. L., Gibbs, N., Waltho, J. P., Clarke, A. R., and Collinge, J. (2001) Proc.Natl.Acad.Sci.U.S.A **98**, 8531-8535
13 Kascsak, R. J., Rubenstein, R., Merz, P. A., Tonna-DeMasi, M., Fersko, R., Carp, R. I., Wisniewski, H. M., and Diringer, H. (1987) J.Virol. **61**, 3688-3693
14 Korth, C., Stierli, B., Streit, P., Moser, M., Schaller, O., Fischer, R., SchulzSchaeffer, W., Kretzschmar, H., Raeber, A., Braun, U., Ehrensperger, F., Hornemann, S., Glockshuber, R., Riek, R., Billeter, M., Wuthrich, K., and Oesch, B. (1997) Nature **390**, 74-77
15 Laffling, A. J., Baird, A., Birkett, C. R., and John, H. A. (2001) Neurosci.Lett. **300**, 99-102
16 Naslavsky, N., Stein, R., Yanai, A., Friedlander, G., and Taraboulos, A. (1997) J.Biol.Chem. **272**, 6324-6331
17 Papa, S., Lorusso, M., and Capitanio, N. (1994) J.Bioenerg.Biomembr. **26,** 609-618
18 Prusiner, S. B. (1998) Proc.Natl.Acad.Sci.U.S.A **95,** 13363-13383
19 Roblero, L., Guadarrama, A., Lopez, T., and Zegers-Hochschild, F. (1996) Reprod.Fertil.Dev. **8**, 871-874
20 Safar, J., Wille, H., Itri, V., Groth, D., Serban, H., Torchia, M., Cohen, F. E., and Prusiner, S. B. (1998) Nat.Med. **4**, 1157-1165
21 Schmerr, M. J. and Jenny, A. (1998) Electrophoresis **19,** 409-414
22 Schreuder, B. E., van Keulen, L. J., Vromans, M. E., Langeveld, J. P., and Smits, M. A. (1996) Nature **381**, 563
23 Shaked, Y., Rosenmann, H., Talmor, G., and Gabizon, R. (1999) J.Biol.Chem. **274**, 32153-32158
24 Shiraishi, N. and Nishikimi, M. (1998) Arch.Biochem.Biophys. **357**, 225-230
25 Stockel, J., Safar, J., Wallace, A. C., Cohen, F. E., and Prusiner, S. B. (1998) Biochemistry **37**, 7185-7193
26 Thackray, A. M., Madec, J. Y., Wong, E., Morgan-Warren, R., Brown, D. R., Baron, T., and Bujdoso, R. (2003) Biochem.J. **370**, 81-90
27 Viles, J. H., Cohen, F. E., Prusiner, S. B., Goodin, D. B., Wright, P. E., and Dyson, H. J. (1999) Proc.Natl.Acad.Sci.U.S.A **96**, 2042-2047
28 Volkel, D., Zimmermann, K., Zerr, I., Bodemer, M., Lindner, T., Turecek, P. L., Poser, S., and Schwarz, H. P. (2001) Transfusion **41**, 441-448
29 Wadsworth, J. D., Joiner, S., Hill, A. F., Campbell, T. A., Desbruslais, M., Luthert, P. J., and Collinge, J. (2001) Lancet **358**, 171-180
30 Weissmann, C. (1996) FEBS Lett. **389**, 3-11
31 Williamson, R. A., Peretz, D., Pinilla, C., Ball, H., Bastidas, R. B., Rozenshteyn, R., Houghten, R. A., Prusiner, S. B., and Burton, D. R. (1998) J.Virol. **72**, 9413-9418

## Claims

1. An immunoassay method for detecting or measuring prion protein in a sample using at least one antibody specifically binding to prion protein, comprising contacting the prion protein present with a reagent capable of improving binding of said at least one antibody to the prion protein.

2. An immunoassay method according to claim 1, wherein said reagent is a metal compound of a prion protein binding metal.

3. An immunoassay method according to claim 2, wherein said metal compound is a salt or chelate of a metal selected from the group consisting of copper, nickel, zinc and manganese.

4. An immunoassay method according to claim 3, wherein said metal salt is selected from the group consisting of CuCl₂, CuSO₄, Cu(NO₃)₂, ZnSO₄, ZnCl₂, Zn(NO₃)₂, NiCl₂, NiSO₄, Ni(NO₃)₂, MnCl₂, MnSO₄ and Mn(NO₃)₂.

5. An immunoassay method according to claim 1, wherein said reagent is an oxidation agent.

6. An immunoassay method according to claim 5, wherein said oxidation agent is hydrogen peroxide or a permanganate salt.

7. An immunoassay method according to any one of claims 1-6, wherein said at least one antibody is a detection antibody.

8. An immunoassay method according to any one of claims 1-7, wherein said prion protein is natural prion protein.

9. An immunoassay method according to any one of claims 1-8, wherein said sample is a body fluid, such as plasma or serum, cerebro spinal fluid, urine, or a tissue homogenate, such as brain homogenate.

10. An immunoassay method according to claim 9, comprising contacting the sample with a solid surface to immobilize prion protein present in the sample to said solid surface, contacting the solid surface, optionally after washing to remove components of the sample which have not been bound to the solid surface, with the reagent, contacting the solid surface, optionally after washing to remove reagent which has not been bound to the solid surface, with a detection antibody specifically binding to prion protein, and determining, optionally after washing to remove detection antibody which has not been bound to the solid surface, the presence or amount of detection antibody which has been bound to the solid surface.

11. An immunoassay method according to claim 10, wherein the immunoassay is in ELISA format.

12. An immunoassay method according to claim 11, wherein the detection antibody used carries a detectable enzyme label and wherein the presence or amount of detection antibody bound to the solid surface is determined by detecting or measuring the conversion of a substrate of said enzyme.

13. An immunoassay method according to claim 11, wherein the solid surface, after said contacting with the detection antibody, is contacted with an antibody specifically binding to the detection antibody and carrying a detectable enzyme label and wherein the presence or amount of detection antibody bound to the solid surface is determined by detecting or measuring the conversion of a measurable substrate of said enzyme.

14. An immunoassay method according to any one of claims 9-13, wherein the prion protein present in the sample is bound by adsorption directly to the solid surface.

15. An immunoassay method according to any one of claims 9-13, wherein the prion protein present in the sample is bound to the solid surface via a substance, such as a peptide or protein, having affinity for prion protein.

16. An immunoassay method according to any one of claims 9-13, wherein the prion protein present in the sample is bound to the solid surface by a catcher antibody specifically binding to prion protein.

17. An immunoassay method according to any one of claims 1-6, wherein said at least one antibody is a catcher antibody.

18. An immunoassay method according to any one of claims 1-8, wherein the sample is a cell or tissue sample, such as brain sample.

19. An immunoassay method according to claim 18, wherein the immunoassay method is in immunohistochemical, immunocytochemical or cytospin format.

20. A method of separating prion protein from a prion protein containing solution using a capturing antibody specifically binding to prion protein, comprising contacting the prion protein present with a reagent capable of improving binding of the capturing antibody to the prion protein.

21. A method according to claim 20, wherein said reagent is a metal compound of a prion protein binding metal, preferably a salt or chelate of a metal selected from the group consisting of copper, nickel, zinc and manganese, such as a metal salt selected from the group consisting of CuCl₂, CuSO₄, Cu(NO₃)₂, ZnSO₄, ZnCl₂, Zn(NO₃)₂, NiCl₂, NiSO₄, Ni(NO₃)₂, MnCl₂, MnSO₄ and Mn(NO₃)₂.

22. A method according to claim 20, wherein said reagent is an oxidation agent, preferably hydrogen peroxide or a permanganate salt.

23. A method according to any one of claims 20-22, wherein the prion protein containing solution after being treated with the reagent is passed through a filter or affinity chromatography column containing capturing antibody immobilized to a solid carrier and wherein the prion protein depleted solution, or the separated prion protein, or both, are collected.

24. A method of determining the nature of prion protein which is present in a sample, comprising the steps of
selecting a reagent capable of improving the binding between prion protein and antibody specifically binding to prion protein,
subjecting the sample to a series of prion protein immunoassays which use the said reagent in various concentrations,
determining the EC₅₀ value of the selected reagent for the prion protein contained in the sample, and
comparing the EC₅₀ value obtained for the prion protein in the sample with the EC₅₀ value of the selected reagent for PrP^{C} and the EC₅₀ value of the selected reagent for PrP^{Sc} to determine the nature of the prion protein contained in the sample.

25. A method according to claim 24, wherein said reagent is a metal compound of a prion protein binding metal, preferably a salt or chelate of a metal selected from the group consisting of copper, nickel, zinc and manganese, such as a metal salt selected from the group consisting of CuCl₂, CuSO₄, Cu(NO₃)₂, ZnSO₄, ZnCl₂, Zn(NO₃)₂, NiCl₂, NiSO₄, Ni(NO₃)₂, MnCl₂, MnSO₄ and Mn(NO₃)₂.

26. A method according to claim 24, wherein said reagent is an oxidation agent, preferably hydrogen peroxide or a permanganate salt.
